# EUROPÄISCHE PATENTANMELDUNG

(11) **EP 0 558 960 A1**
(43) Veröffentlichungstag der Anmeldung: **08.09.1993**
(21) Anmeldenummer: 93102029.1
(22) Anmeldetag: 10.02.1993
(51) Int. Cl.: A61K 31/68, A61K 31/51, A61K 31/505

(54) **Arzneimittel zur Prophylaxe und Therapie von neurologischen und psychiatrischen Schäden durch Alkoholmissbrauch, enthaltend Thiamin, Folsäure und Cobalamin**

(30) Priorität: 29.02.1992 DE 4206422
(71) Anmelder: WÖRWAG PHARMA GmbH, D-70499 Stuttgart (DE)
(72) Erfinder: Loew, Dieter, Prof. Dr. Dr., W-5600 Wuppertal (DE); Haller, Claus-Peter, Dr., W-7016 Gerlingen (DE); Wörwag, Fritz, Dr., W-7000 Stuttgart 1 (DE)
(74) Vertreter: Weller, Wolfgang, Dr.rer.nat.

(57) **Zusammenfassung**

Ein Arzneimittel zur Prophylaxe und Therapie von neurologischen und psychiatrischen Schäden durch Alkoholmißbrauch weist als Wirkstoffe Thiamin, Folsäure und eine deren biologische Verwertbarkeit ermöglichende Menge an Cobalaminen und/oder pharmakologisch gleichwirkende Analoge der vorgenannten Stoffe in einem pharmazeutisch verträglichen Träger auf.

## Beschreibung

Die Erfindung betrifft ein Arzneimittel zur Prophylaxe und Therapie von neurologischen und psychiatrischen Schäden durch Alkoholmißbrauch.

Die neurologischen und psychiatrischen Schäden bei Alkoholmißbrauch sind Konsequenz einer direkten Wirkung des Alkohols auf das Zentralnervensystem. Als Folge eines akuten oder chronischen Alkoholmißbrauchs stellen sich unterschiedliche neurologische Erkrankungen und Symptome ein. Ursachen sind alkoholbedingte Stoffwechselstörungen und toxische Abbauprodukte des Alkohols. Aus der alkoholbedingten Resorptionsstörung von B-Vitaminen im Magen-Darm-Trakt einerseits, den alkoholbedingten Stoffwechselstörungen sowie der toxischen Wirkung seiner Metaboliten andererseits, resultieren letztendlich Organ- und Enzymstörungen, die sich in dem charakteristischen Bild der alkoholischen Polyneuropathie, der Wernicke-Enzephalopathie und im Korsakow-Syndrom äußern.

Aus zahlreichen Untersuchungen der letzten Jahrzehnte geht hervor, daß bei chronischem Alkoholismus ein Mangel an Vitamin B₁, also an Thiamin und auch an Folsäure besteht:
Bachevalier, J., Joyal, C., Botez, M.I. Blood Thiamine and blood folate levels. A comparative study in control, alcoholic and folate-deficient subject Int. Zschr.Vit.- und Ernähr.Forsch. 51 (1981), 205-210
Baker, H.
A Vitamin profile of Alcoholism.
in: Vitamins in Medicin (Ed.A.Hank), Huber, Bern-Stuttgart-Wien 1983, 179-184
Berthier, C., Barnaud, J., Bonneru-Gouchoud
Interet de la Vitamin B₁ haute dose pour. Le traitement des encephalopathies alcooliques. A propos de 3 abservations Le Journal de Mediciene de Lyon 38 (1957), 421-424
Leevy, C.M.
Thiamin deficiency and alcoholism.
in Thiamin: Twenty years of progress.
Eds.Sable, H.Z. Gubler, C.J., The New York Academy of Sciences, 1982, 316
Thomson, A.D., Ryle, P.R., Shaw, G.K.
Ethanol, Thiamine and brain damage.
Alcohol & Alcohlism 18 (1983), 27-43
Monographie zu Thiamin
Bundesanzeiger Nr. 131 vom 21.07.1987
Monographie zu Folsäure
Bundesanzeiger Nr. 45 6.3.1987
Scott, H.M., Dinn J.J. Wilson, P., Weir. D.G.
Pathogenesis of subacute combined degeneration: A result of methyl group deficiency
Scott, J.M., Weir, D.G.
The Methyl Folate Trap
Lancet 2 (1981), 337-340
Thiamin und/oder dessen biologisch gleichwirkende Analoge, die auch unter dem allgemeinen Begriff Vitamin B₁ zusammengefaßt werden, und Folsäure, früher auch Vitamin B₉ oder auch Vitamin M genannt, sind essentielle Wirkstoffe, auf die der Organismus wegen fehlender Eigensynthese für einen geregelten Ablauf verschiedener Stoffwechselprozesse angewiesen ist. Obwohl bei ausgewogener Ernährung ausreichend Vitamine aufgenommen und in bestimmten Ausmaß gespeichert werden, können aus einer gestörten Resorption oder einem erhöhten Umsatz Vitamin-abhängige Störungen und Krankheiten auftreten. Zur Prophylaxe und zur Therapie von neurologischen und psychiatrischen Schäden durch Alkoholmißbrauch wurde daher die Verabreichung von Thiamin, insbesondere in Form von Thiamin-Hydrochlorid vorgeschlagen (siehe Monographie zu Thiamin, a.a.O.).

Es wurde nunmehr festgestellt, daß zwar Folsäure gut und rasch vom menschlichen Körper resorbiert wird und sich entsprechend rasch im Körper verteilt, daß jedoch die überwiegende Menge an zugesetzter Folsäure vom Körper nicht biologisch verwertet wird. Es wurde außerdem festgestellt, daß durch eine erhöhte Folsäureaufnahme Schädigungen am menschlichen Körper auftreten können (siehe insbesondere Monographie zu Folsäure, a.a.O.).

Es ist daher Aufgabe der vorliegenden Erfindung, ein Arzneimittel der eingangs erwähnten Art zu schaffen, das eine biologisch verwertbare Zuführung der Wirkstoffe zur Behandlung der Schäden durch Alkoholmißbrauch in den menschlichen Körper ermöglicht.

Erfindungsgemäß wird die Aufgabe dadurch gelöst, daß als Wirkstoffe Thiamin sowie Folsäure und eine deren biologische Verwertbarkeit ermöglichende Menge an Cobalaminen in einem pharmazeutisch verträglichen Träger enthalten sind.

Cobalamine, die auch als Vitamin B₁₂ bezeichnet werden, stehen in einem Wirkungssynergismus mit Folsäure. Das bedeutet, Folsäure ist nur biologisch verwertbar, wenn gleichzeitig Cobalamine zur Verfügung stehen, da beide Substanzen an der enzymatischen Methionin-Synthetase-Reaktion beteiligt sind. Bei diesem Stoffwechselschritt erfolgt der Transfer der Methylgruppe von Methyltetrahydrofolsäure auf Homocystein unter Bildung von Methionin. Bei einem Cobalaminmangel ist diese Reaktion aufgrund eines Cofactor-defizits blockiert, woraus eine Verarmung des Organismus an reaktionsfähigen Folatverbindungen resultiert. Als Folge der Akkumulation von N-5-Methyl-Tetrahydrofolsäure resultieren bei einem Cobalaminmangel erhöhte Folsäure-Konzentrationen im Plasma und erniedrigte Folat-Konzentrationen in den Erythrozyten, da keine Tetrahydrofolsäure aus 5-Methyl-Tetrahydrofolsäure für die Synthese der speicherfähigen Folat-Polyglutamat-Verbindungen zur Verfügung gestellt wird.

Die nunmehr vorgeschlagene Zugabe von Cobalaminen, obwohl festgestellt wurde, daß durch Alkoholmißbrauch ein Mangel an diesen Substanzen nicht hervorgerufen wird, oder ein tatsächlich vorhandener Mangel nicht ursächlich für die neurologischen und psychiatrischen Schäden ist, hat nun den erheblichen Vorteil, daß der zur Prophylaxe oder Behandlung verabreichten Folsäure zugleich deren Wirkungspartner zugegeben wird, der erforderlich ist, um die Folsäure biologisch verwertbar zu machen. Durch die Hinzugabe einer solchen Menge an Cobalaminen, die die biologische Verwertbarkeit der Folsäure sicherstellt, ist die Behandlung unabhängig davon, ob eine ausreichende Menge an Cobalaminen bei dem zu behandelnden Patienten vorhanden ist oder nicht. Ferner ist sichergestellt, daß mit der Verabreichung der Folsäure, die wie zuvor erwähnt, sehr rasch vom Körper resorbiert und verteilt wird, zugleich deren Wirkungspartner vorhanden ist. Es können daher die beobachteten Schäden, die bei einer erhöhten Verabreichung von Folsäure alleine auftreten, von vorneherein ausgeschlossen werden. Dadurch können auch Schädigungen durch Überdosen des Arzneimittels ausgeschlossen werden. Dies berücksichtigt die besondere Patientengruppe der Alkoholgeschädigten, die im Rahmen der Behandlung ihrer Krankheit alkoholbedingt Phasen durchschreiten, die deren Handlungsfähigkeit stark einschränken, so daß die Gefahr besteht, daß gegenüber Dosierungsvorgaben von Arzneimitteln sorglos umgegangen wird. Aufgrund der Tatsache, daß Folsäure mit einer entsprechenden Menge an Cobalamin zugleich vorhanden ist, kann Folsäure auch bei hoher Verabreichung abgebaut werden, ohne daß Schädigungen durch hohe Konzentrationen an Folsäure im Körper überhaupt auftreten können.

Ein weiterer Vorteil der Zugabe des Cobalamins ist, daß für die Resorption von Cobalaminen ein aktiver intrinsic-factorabhängiger Mechanismus und eine passive Diffusion besteht. Als sogenannter extrinsic-factor bildet das Cobalamin mit dem von der Magenschleimhaut gebildeten intrinsic-factor einen Komplex, der im unteren Teil des Dünndarmes (Ileum) resorbiert wird. Aufgrund dieses besonderen Resorptionsmechanismus wird nur so viel Cobalamin aufgenommen, wie der Organismus benötigt. Somit wird durch die jetzt vorgeschlagenen Maßnahmen erreicht, daß indirekt über den Wirkungssynergismus mit Folsäure, einerseits eine ausreichende Menge an Cobalaminen vorhanden ist, um die biologische Verwertbarkeit der zugeführten Folsäure sicherzustellen, daß aber andererseits eben zugleich auch nur die dazu benötigte Menge an Cobalaminen vom Körper resorbiert wird.

Daraus resultiert quasi eine doppelte Sicherheit, d.h., daß zum einen gewährleistet ist, daß der resorbierten Folsäure der Wirkungssynergismuspartner zur Verfügung steht, so daß keine schädlichen Folsäureüberkonzentrationen entstehen können, andererseits aber auch eben nur die Mengen resorbiert werden, die der Körper benötigt.

Der weitere Wirkstoff Thiamin trägt in Kombination mit den zuvor genannten Wirkstoffen besonders wirkungsvoll zur Behebung der bei Alkoholikern beobachteten Polyneuropathien bei.

Es wurde festgestellt, daß in der vorgeschlagenen Kombination die Substanzen Thiamine, Folsäure und Cobalamine eine sehr gute Bioverfügbarkeit aufweisen, die bei verschiedenen Applikationsformen jeweils gleich gut ist. Ein Maß dafür ist der Zeitpunkt der maximalen Serumkonzentration Cₘₐₓ der Wirkstoffe. Es wurde nun überraschend festgestellt, daß auch bei verschiedenen Applikationsformen der Zeitpunkt der maximalen Serumkonzentration, also das Maximum der im Serum nachgewiesenen einzelnen Wirkstoffe jeweils gleich ist. Die Wirkstoffkombination ist somit im Körper zeitlich gleich bioverfügbar und kann daher zusammenwirkend zur Prophylaxe und Therapie von neurologischen und psychiatrischen Schäden durch Alkoholmißbrauch eingesetzt werden. Die Untersuchungen ergaben, daß bei Applikation des Arzneimittels in wässriger Lösung oder als Vitaminkapsel das Serummaximum nach etwa 1 bis 1 1/2 Stunden erreicht wurde, so daß neben der jeweils gleichzeitigen Bioverfügbarkeit der Einzelsubstanzen auch eine sehr rasche Bioverfügbarkeit vorhanden ist, wobei lediglich der absolute Zeitpunkt bei verschiedenen Applikationsformen der Kombination unterschiedlich ist. Somit ist es auch möglich, in akuten Fällen über von Patienten problemlos anwendbaren Applikationsformen, wie Trinklösungen, Tropfen oder Kapseln rasche Linderung zu besorgen. So äußern sich akute Erscheinungen von neurologischen Schäden durch Alkoholmißbrauch in starkem Kribbeln in den Extremitäten, das durch Einnahme von Kapseln in kurzer Zeit beseitigt werden kann. Untersuchungen haben gezeigt, daß mit der Verabreichung der jetzt vorgeschlagenen Wirkstoffkombination erfolgreich typische Symptome der Schädigung durch Alkoholmißbrauch beseitigt werden können.

Als pharmakologisch gleichwirkende Analoge der Wirkstoffe können Substanzen eingesetzt werden, die chemisch abgewandelt sind, die jedoch vom Körper wieder in die Wirkstoffe Thiamin, Folsäure oder Cobalamine umgesetzt werden. Es sind als pharmakologisch gleichwirkende Analoge auch die Stoffwechselmetaboliten der Wirkstoffe einzusetzen, da die Wirkstoffe Thiamin, Folsäure und Cobalamine selbst im Stoffwechselvorgang (Metabolismus) unter Durchschreiten abgewandelter Formen (Metaboliten) umgesetzt werden. So kann es in besonders akuten Fällen geboten sein, beispielsweise anstatt der Folsäure bereits deren Metabolit Tetrahydrofolsäure einzusetzen, der in einem ersten Stoffwechselschritt aus der Folsäure vom Körper gebildet wird. Bei der Zugabe von Tetrahydrofolsäure anstatt Folsäure kann dann der Zeitraum eingespart werden, den der Körper benötigt, um die zugeführte Folsäure in Tetrahydrofolsäure umzuwandeln. Dies ist beispielsweise auch bei den Thiaminen möglich, d.h. es können bereits Thiamintriphosphat oder Thiaminpyrophosphat eingesetzt werden.

Das Einsetzen von chemisch abgewandelten Wirkstoffen dient dazu, beispielsweise die Lagerfähigkeit oder Haltbarkeit der Wirksubstanzen zu erhöhen, oder deren Eignung für bestimmte Applikationsformen zu verbessern. So können beispielsweise hydrophile Gruppierungen die Wasserlöslichkeit verbessern. Diese Gruppierungen werden vom Organismus abgespalten und die Wirkstoffe werden in ihre Ausgangsformen des Metabolismus, also in Thiamin, Folsäure oder Cobalamin umgewandelt.

Somit wird die Aufgabe vollkommen gelöst.

In besonders vorteilhaften Ausgestaltungen der Erfindung wird dazu neben Thiamin, dessen Hydrochlorid oder entsprechende Salze als Wirkstoff Benfotiamin, Acetiaminhydrochlord, Bentiaminlaurilsulfat, Bisbentiamin, Fursultiamin, Fursultiaminhydrochlorid, Thiamindihydrogenphosphatchlorid, Thiamindihydrogenphosphatdihydrogenphosphat, Thiaminnitrat, Vitamin B₁, Octotiamin, Thiamindisulfid, Thiamindisulfid-O-O'-dinicotinat eingesetzt.

Als Thiaminmetaboliten werden Thiaminnitrophosphat (TTP)oder Thiaminpyrophosphat (TPP) eingesetzt.

In einer besonders vorteilhaften Ausgestaltung sind lipoidlösliche Thiamine insbesondere aus der Gruppe der Allithiamine enthalten.

Als Metaboliten der Folsäure sind besonders Tetrahydrofolsäure oder Folinsäure geeignet.

Als Cobalaminderivate sind insbesondere Cyanocobalamin, Hydroxocobalamin, Methylcobalamin oder die Metaboliten Methylcobalamin, 5-Adenosylcobalamin geeignet.

Als besonders vorteilhaft hat sich Benfotiamin herausgestellt, das besser verfügbar als Thiaminhydrochlorid ist und im Gegensatz zu Thiaminhydrochlorid sogar die Blut-Hirn-Schranke passiert und sich im Liquor cerebrospinalis anreichert, was ein wichtiger Gesichtspunkt in der Behandlung des Wernicke und Korsakow-Syndromes ist.

So wurde festgestellt, daß bei oraler Verabreichung des erfindungsgemäßen Arzneimittels mit dem Wirkstoff Benfotiamin 10-fach höhere Plasmaspiegel an Thiamin und ca. 130-fach höhere Anstiege von Thiamin und Thiamindiphosphat-coenzymen in den Erythrozyten vorliegen.

Besonders bevorzugt ist ein Arzneimittel, das als Wirkstoff Benfotiamin, Folsäure und Cyanocobalamin enthält.

In dieser Ausgestaltung liegt dem ersten Wirkstoff des Arzneimittels, nämlich der Folsäure, der Wirkungspartner Cyanocobalamin in der zur Bildung des Komplexes im unteren Teil des Ileums günstigsten Resorptionsform vor, und zugleich liegt der zweite Wirkstoff des Arzneimittels in der günstig resorbierbaren lipoidlöslichen Form, nämlich als Benfotiamin vor.

Die Wirkstoffe können pro Dosis in Mengen von 1mg - 500mg Thiamin, insbesondere Benfotiamin; 0,1mg - 50mg Folsäure und 1mcg - 5mg Cobalamin enthalten sein.

In diesen Mengenverhältnissen ist eine optimale biologische Verwertbarkeit der Wirkstoffe des Arzneimittels vom Körper möglich.

Die Darreichungsformen des Arzneimittels bestehen besonders vorteilhaft in oralen Darreichungsformen, beispielsweise in Form von Kapseln, Dragees, Tabletten, Filmtabletten, Lutsch-, Kau-, Brausetabletten, Trinklösungen oder Tropfen. Es ist auch möglich, das Arzneimittel in parenteralen Darreichungsformen z.B. in Form von Injektionslösungen oder Infusionslösungen oder in rektalen Darreichungsformen wie z.B. Zäpfchen sowie in nasalen Darreichungsformen zu verabreichen. Es ist auch möglich, das Arzneimittel in Form von Inhalationen zu verabreichen.

Es sind daher alle gebräuchlichen Darreichungsformen möglich, so daß bei der problematischen Gruppe von Alkoholgeschädigten jeweils dem Patienten angenehmste oder verabreichbare Applikationsformen ausgewählt werden können.

Es versteht sich, daß die vorstehend genannten und die nachstehend noch zu erläuternden Merkmale nicht nur in den angegebenen Kombinationen, sondern auch in Alleinstellung in anderen Kombinationen einsetzbar sind, ohne den Rahmen der vorliegenden Erfindung zu verlassen.

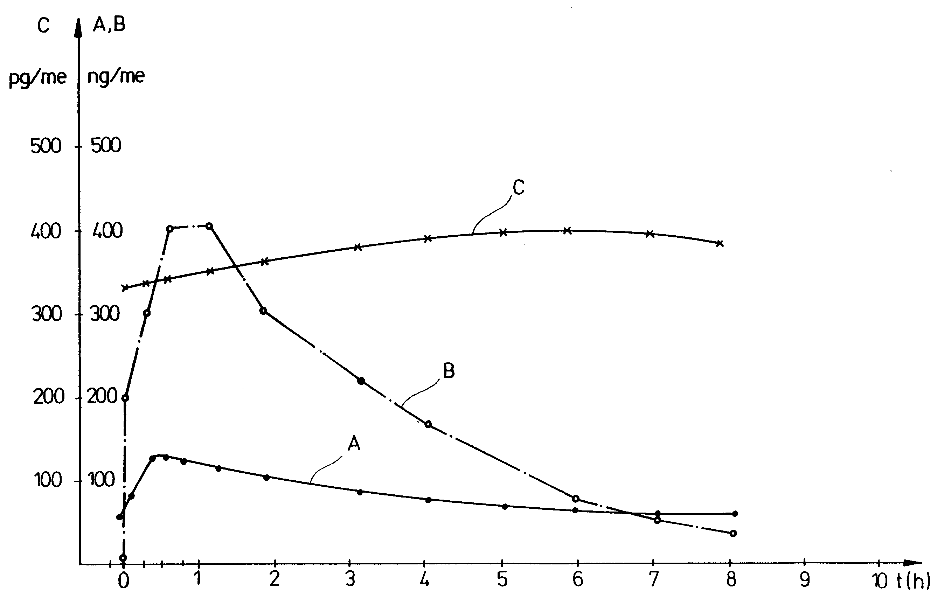

Die Erfindung wird nachfolgend im Zusammenhang mit der beiliegenden Zeichnung näher beschrieben und erläutert. Die einzig beiliegende Zeichnung ist ein Diagramm, das den zeitlichen Verlauf der Serumkonzentrationswerte der einzelnen Wirkstoffe darstellt.

In einer Studie mit 12 Probanden (6 Frauen und 6 Männer) wurde die Bioverfügbarkeit von Folsäure, Cobalamin und Benfotiamin aus verschiedenen Zubereitungen (Kapsel/wässrige Lösung) ermittelt.

Unter Einhaltung standardisierter Rahmenbedingungen wurden die jeweiligen Zubereitungen im cross-over-design verabreicht. Zur Bestimmung wurden jeweils unmittelbar vor, sowie 15, 30, 45, 60, 90 und 120 Minuten und nachfolgend in 2stündigen Abständen bis zur 10. Stunde nach der Einnahme der Präparate Blutproben entnommen. Die Analytik erfolgte radioimmunologisch (Folsäure, Cobalamin) bzw. hochdruckflüssigkeitschromatographisch (Thiamin). Die Prüfpräparate wurden als Vitamin-Kapseln oder als wässrige Lösung verabreicht.

| Vitamin-Kapsel: | |
|---|---|
| Folsäure | 10,0 mg/Kapsel |
| Cyanocobalamin | 0,3 mg/Kapsel |
| Benfotiamin | 50,0 mg/Kapsel |

| Wässrige Lösung: | |
|---|---|
| Folsäure | 10,0 mg |
| Cyanocobalamin | 0,3 mg |
| Benfotiamin | 50,0 mg. |

Im beiliegenden Diagramm sind die Konzentrationen im Plasma dargestellt. Dabei sind die arithmetischen Mittelwerte aus den an 12 Probanden gewonnenen Meßergebnissen aufgetragen. Auf dem Zeitstrahl bedeutet der Zeitpunkt t=0 der Zeitpunkt der ersten Messung 15 Minuten nach Verabreichung der Wirkstoffkombination in Form einer wässrigen Lösung.

Die Kurve A stellt den Konzentrationsverlauf von Thiamin im Plasma dar, und zwar in ng/ml.

Die Kurve B zeigt den Konzentrationsverlauf der Folatkonzentration im Plasma an, und zwar in ng/ml.

Die Kurve B zeigt den Konzentrationsverlauf von Cobalamin im Plasma und zwar in pg/ml.

Aus der Figur ist zu entnehmen, daß die maximale Konzentration im Plasma sowohl von Thiamin als auch von Folat nach etwa einer halben Stunde nach Applikation erreicht ist. Somit stehen beide Wirksubstanzen bereits nach kurzer Zeit in ihrem maximalen Konzentrationsmaß zur Verfügung, können also gleichzeitig ihre Wirkung entfalten. Somit liegt eine sehr rasche und gleichzeitige Bioverfügbarkeit der Wirksubstanzen vor.

Bei der Verabreichung in Form der Vitamin-Kapsel ist das Maximum der Kurven A und B zeitlich etwa um 15 bis 30 Minuten später zu beobachten. Aus der Kurve C, der Cobalamin bzw. Vitamin B12-Konzentration im Plasma ist zu entnehmen, daß dieses eine Steuerfunktion über die Aufnahme und Verwertung des Synergiepartners Folsäure durchführt, d.h. es wird nur eine bestimmte Menge, jedoch etwa konstant ansteigend im Plasma beobachtet. Dies läßt auf einen Intrinsikfaktor gesteuerten Resorptionsmechanismus schließen, d.h. es wird dem Körper eine ausreichende gesteuerte Menge an Folsäure zugeführt, die jedoch überraschenderweise zeitgleich bioverfügbar mit Thiamin ist, somit rasch und gleichzeitig die Kombinationswirkung im Organismus ausgeübt wird.

Probanden mit akuten Symptomen von durch Alkohol bedingten Schäden, beispielsweise mit extremem Kribbeln in den Extremitäten zeigten bereits wenige Minuten nach Verabreichung der Wirkstoffkombination beträchtliche Linderung dieser Symptome.

Weitere Untersuchungen haben gezeigt, daß Erscheinungen wie Konzentrationsmangel, mangelnde Merkfähigkeit und eingeschränkte Artikulationsfähigkeit wesentlich gelindert werden konnten.

## Patentansprüche

1. Arzneimittel zur Prophylaxe und Therapie von neurologischen und psychiatrischen Schäden durch Alkoholmißbrauch, dadurch gekennzeichnet, daß als Wirkstoffe Thiamin, Folsäure und eine deren biologische Verwertbarkeit ermöglichende Menge an Cobalaminen und/oder pharmakologisch gleichwirkende Analoge der vorgenannten Stoffe in einem pharmazeutisch verträglichen Träger enthalten sind.

2. Arzneimittel nach Anspruch 1, dadurch gekennzeichnet, daß als Thiamin und/oder dessen pharmakologisch gleichwirkende Analoge Wirkstoffe ausgewählt aus der Gruppe bestehend aus Thiaminchlorid, Thiaminhydrochlorid, Benfotiamin, Acetiaminhydrochlorid, Bentiaminlauril-sulfat, Bisbentiamin, Fursultiamin, Fursultiamin-hydrochlorid, Thiamindihydrogenphosphatchlorid (Estersalz), Thiaminhydrogenphosphatdihydrogenphosphat (Estersalz), Thiaminnitrat, Vitamin B₁, Octotiamin, Thiamindisulfid, Thiamindisulfid-O,O'-dinicotinat enthalten sind.

3. Arzneimittel nach Anspruch 1, dadurch gekennzeichnet, daß als pharmakologisch gleichwirkende Analoge des Thiamins stoffwechselaktive Metaboliten des Thiamins eingesetzt werden.

4. Arzneimittel nach Anspruch 3, dadurch gekennzeichnet, daß als Metaboliten des Thiamins Thiamintriphosphat (TTP) oder Thiaminpyrophosphat (TPP) enthalten sind.

5. Arzneimittel nach einem der Ansprüche 1 bis 3, dadurch gekennzeichnet, daß lipoidlösliche Thiamine, nämlich die Gruppe der Allithiamine enthalten sind.

6. Arzneimittel nach einem der Ansprüche 1 bis 5, dadurch gekennzeichnet, daß als pharmakologisch gleichwirkende Analoge der Folsäure Folsäurederivate oder Folsäuremetaboliten ausgewählt aus der Gruppe bestehend aus Tetrahydrofolsäure, Folinsäure enthalten sind.

7. Arzneimittel nach einem der Ansprüche 1 bis 6, dadurch gekennzeichnet, daß als Cobalamine Stoffe ausgewählt aus der Gruppe bestehend aus Cyanocobalamin, Hydroxocobalamin, Methylcobalamin, 5-Adenosylcobalamin enthalten sind.

8. Arzneimittel nach einem der Ansprüche 1 bis 7, dadurch gekennzeichnet, daß die Wirkstoffe pro Dosis in Mengen von
1 mg - 500 mg Thiamin,
0,1 mg - 50 mg Folsäure, und
1 mcg - 5 mg Cobalamin
enthalten sind.
